(19) Europäisches Patentamt
European Patent Office
Office européen des brevets

(11) **EP 4 696 336 A1**

(12) **EUROPEAN PATENT APPLICATION**

(43) Date of publication:
**18.02.2026 Bulletin 2026/08**

(21) Application number: **25195556.3**

(22) Date of filing: **12.08.2025**

(51) International Patent Classification (IPC):
**A61L 17/04** $^{(2006.01)}$      **A61L 17/14** $^{(2006.01)}$

(52) Cooperative Patent Classification (CPC):
**A61L 17/04; A61L 17/145;** A61L 2300/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **15.08.2024 US 202418831138**

(71) Applicant: **Roshkovan, Igor**
**Los Angeles, CA 90024 (US)**

(72) Inventor: **Roshkovan, Igor**
**Los Angeles, CA 90024 (US)**

(74) Representative: **Margue, Robert Germain**
**Straßbergerstraße 139 / 45**
**80809 München (DE)**

(54) **NON-ABSORBABLE SURGICAL BIO-SUTURE MATERIAL MADE FROM AGAVE AMERICANA PLANT LEAF FIBERS USING A POTASSIUM PERMANGANATE COATING**

(57)    A surgical suture includes an elongated core comprising a plurality of bundles of bio-composites of fibers made from Agave americana plant leaf. A surgical bio-suture material core from Agave americana plant fibers has two ends and one end combined with a surgical needle.

**Fig. 4**

EP 4 696 336 A1

**Description**

**BACKGROUND- PRIOR ART**

The present inventions related to medical devices such as surgical suture material used in surgery, in particular to general surgery, orthopedic surgery, plastic and reconstructive surgery, or dental surgery and veterinary surgery. More particularly, the invention relates to non-absorbable surgical bio-composite sutures that are made from Agave americana plant leaf fibers using potassium permanganate coating which can be used as a material potential alternative for non-absorbable materials such as Silk, Polypropylene sutures, Nylon (polyamide), Polyester, and PVDF sutures and a method of producing it.

This disclosure concerns improved health care and dental care for all patients who had surgical intervention, and more to a novel method of using a non-absorbable surgical bio-suture material made from Agave americana plant leaf fibers using potassium permanganate coating and a method of producing it.

These inventions herein refer to a novel surgical bio-suture material, to a technique of making the suture material made from Agave americana plant leaf using potassium permanganate coating. More specifically, these inventions relate to a new approach of a non-absorbable surgical suture material made from the Agave americana plant leaf using potassium permanganate coating and a method of producing it.

The present invention for suture material was created through the improvement of methods of manufacturing the current suture materials.

This disclosure concerns improved dental care for dental patients, and more to a novel non-absorbable surgical suture material made from Agave Americana plant leaf using potassium permanganate coating. This embodiment relates particularly to suture material devices having improved function to reduce hemorrhage reaction, tissue sensitivity, wound edges dehiscence, and wound infection.

Sutures are a medical device, that is used to join the soft tissue, skin, and blood vessels, and bring together the tissues for the healing process in surgery. Sutures need to stay in place for a specific period and stimulate healing until the primary tension occurs.

Suturing has been used over the centuries to help human tissues heal, by approximating the wound edges and reducing the open surgical space.

Surgical sutures are widely used in the medical field, specifically in a wide range of general surgeries, or dental or veterinary surgical procedures to close wounds, hold soft tissues together or mucosa, and stimulate healing.

Surgical sutures are classified into non-absorbable and absorbable sutures. The surgical suture materials can be mono-filament, multi-filament, or braided.

Absorbable sutures are absorbed by soft tissue or saliva, making removal avoidable. A well-known suture made of catgut is an example of an absorbable suture material. The non-absorbable sutures are not absorbed. They can be removed by healthcare practitioners. Established materials for non-absorbable sutures are silk, nylon (polyamide), polypropylene, and PTFE monofilament. Non-absorbable sutures are mainly used for general surgery and oral surgeries. In the meantime, absorbable sutures are mainly used for plastic surgery, fixing of skin, and suturing of soft tissue.

In addition, there are other mechanical wound closure medical devices available such as staples, tapes, and adhesives, but surgical sutures are still the most popular.

Surgical sutures are widely used in the medical field, specifically in a wide range of general surgeries, to close wounds, hold soft tissues together, and stimulate healing. Suture materials are necessary in every surgical procedure, for the repair of incised damaged soft tissues.

In dentoalveolar surgery, suture material can be used to re-approximate soft tissues separated by surgical procedure or accidental trauma, to promote the primary healing process and to control hemorrhage.

Suture materials are characterized by numerous methods implying mechanical and physical properties, handling characteristics, and biological and biodegradation performance. In most surgical cases silk sutures are used to suture the mucosal tissues. Silk suture material has low tensile strength, breaking under high tension. Nylon suture is the most commonly used in cardiovascular, neurological, and ophthalmic neurological surgeries. The nylon has high tensile strength but loses it over time. Nylon is stiff, making handling and tying very difficult. They are also a source of surgical infections and healing complications.

While little clinical data exists to guide the choice of suture material, this choice for health practitioners is a crucial one as it may play a major role in wound infection. The wound infection is usually detected when the wound contains purulent material or shows additional clinical signs of infection such as warmth, erythema, and local tenderness.

In addition, if the suture materials are lost too early, wound dehiscence may occur, delaying and interrupting the healing process. The major disadvantage of suture material is making knot security poor.

In a clinical where the practitioner uses silk sutures that have not been removed and sunk deeply into the alveolar mucosal tissue, forgotten silk sutures may cause irritation and pain to patients. Moreover, if suture material is retained too long, it can cause inflammation and may lead to granuloma formation or abscess. However, as the wound area heals, the suture material will remain loose, and it accommodates tissue swelling. If the suture materials are lost too early, wound dehiscence may occur, delaying and interrupting the healing process. All suture materials without exception can retain bacteria colonies that may result in infection and may be a harbor for bacteria and biofilms. They are also a source of surgical infections and post-operative healing complications. Using medical irrigation, clinicians try to completely eliminate bacteria and biofilm in surgical areas but without success.

The most common complications for sutures are hemorrhage, tissue reactivity, wound edges dehiscence, and wound infection.

The following is a tabulation of some prior art that presently appears relevant:

**U.S. Patents:**

| Pat. No | Kind C ode | Issue Date | Patentee |
|---|---|---|---|
| US3716058 | A | 1973 -02-13 | J. Tanner |
| US9248580 | B2 | 2016-02-02 | J. Leung |
| US9675341 | B2 | 2017-06-13 | W. D'Agostino |
| US10178991 | B2 | 2019-01-15 | P. Bailly |
| US10492780 | B2 | 2019-12-03 | J. Gross |
| US11007296 | B2 | 2021-05-18 | J. Gross |
| US9248580 | B2 | 2016-02-02 | J. Leung |
| USRE45426 | E1 | 2015-03-17 | H. Buncke |
| US4259959 | A | 1981-04-07 | W. Walker |
| US9848868 | B2 | 2017-12-26 | J. Saliman |
| US8361112 | B2 | 2013-01-29 | K. Carroll |
| US7815662 | B2 | 2010-10-19 | J. Spivey |
| US8784438 | B2 | 2014-07-22 | T. Mikkaichi |

**Foreign Patent Documents:**

| Foreign Doc. Nr. | Cntry. Code | Kind Code | Pub. Dt. | Patentee |
|---|---|---|---|---|
| WO1992007519 | GE | A1 | 1992-05-14 | M. Neumann |

The primary purpose of suturing is to bring two edges of soft tissues together, keeping them in place until they heal and join together.

The current inventions relate to a non-absorbable suture built up of an Agave americana plant fiber using potassium permanganate coating. Agave is scientifically classified as being from the Plantae Kingdom, the Tracheophytes, Angiosperms, and Monocots Clades, the Asparagales Order, the Asparagaceae Family, and the Agavoideae subfamily. The specific species includes Agave americana. Agave americana, commonly known as the century plant, maguey, is a flowering plant species. It is native to Mexico and the United States, precisely Texas and California.

Synthetic suture material is disreputable for causing substantial foreign body reactions of soft tissues and having extensive degradation outlines that continue longer after wound healing has been completed.

The future manifestation of potential infection should be strongly respected when choosing the appropriate suture type material. The suture materials perform differently with contaminated wounds after surgery. Clinicians know that in the face of infection, any retained sutures may harbor bacterial infection. Furthermore, some sutures are more liable to biofilm created by bacteria development in the presence of infection.

Surgical sutures are commonly used in all types of surgeries with the presence of bacterial contamination. Acute or chronic surgical site infections such as the bacterial load frequently accompany suturing. The bacterial loading in the surgical area leads to the appearance of necrotic soft tissue and the stimulation of the process of colonization of the bacteria mass and the stimulation of the adherent bacteria and biofilms that are attached to the sutures. Suture materials surrounded by biofilms may increase the risk of persistent acute or chronic infection, surgical revision, and morbidity.

Generally, the majority of suture materials in the market have less favorable strength-retention characteristics.

As a result, we need surgical bio-suture material with several beneficial features such as the lowest level of microbial growth and the least scar formation. There is a need to greatly minimize trauma and discomfort to patients.

**BRIEF SUMMARY AND OBJECTS OF THE EMBODIMENT**

In reaction to the complications and problems discussed herein, very effective surgical bio-sutures from Agave americana plant leaf fibers are provided.

A novel biomaterial suture that is, monofilament and efficient of drug release, was developed from a combination of natural bio fibers that were not previously applied for this purpose. Herein, we define a novel suture material that consists of certain organic chemicals that provide antibacterial properties that not only meet the United States Pharmacopeia's requirements for suture tensile strength but also act as a contained drug release device.

Naturally derived fibers have excellent biocompatibility, impressive biodegradation properties, and are similar to the native extracellular matrix. The bio-suture material must be hinged not only on the well-known good properties of the bio-organic chemicals with specific properties such as non-toxic, biocompatible, biostable, biodegradable, and hydrophilic. Furthermore, bio-suture materials as drug release devices present several unique benefits that include localized drug delivery, which avoids the risk of local side effects and lowers the amount of drug needed.

Fibers extracted from the leaf of the Agave americana plant are cellulosic in nature and have unique properties such as anti-inflammatory, immune stimulation, antioxidant, antimicrobial, and antifungal activities. Moreover, Agave americana leaf fibers using potassium permanganate coating are a valuable source of bio-active complexes that have health benefits. In addition, the Agave leaf fibers have a significant saponin content, phenolic compounds, and terpenes which are primary phytochemicals found in Agave americana leaf. Various of the beneficial health properties assigned to Agaves americana fiber saponins comprise anti-inflammatory and immune stimulation activities.

The health benefits such as the antioxidant effects of Agave americana leaf fibers are associated with the presence of flavonoids such as quercetin and kaempferol. The antimicrobial and antifungal impacts of Agave americana leaf fibers are also linked to phenolic compounds and saponins, which interact with bacterial membrane proteins and lipids to cause bacteria membrane integrity leakage of intracellular components in Gram-positive and Gram-negative bacteria. Besides, the Agave americana leaf fibers have an inhibitory effect on microorganisms, such as Escherichia coli, Staphylococcus aureus, Pseudomonas aeruginosa, Bacillus cereus, Listeria monocytogenes, and Clostridium perfringens.

Additionally, braided suture materials appear to provide a more favorable bacterial environment compared with monofilament suture material, especially in observing bacterial adherence to sutures.

Generally established characteristics of mechanical properties of an ideal suture material consider good tensile strength, superior knot security, percentage elongation, modulus of elasticity, stress relaxation, and creep. The ideal suture material also has excellent handling characteristics, minimal tissue reaction, absence of allergy reactions, and resistance to infection.

Agave americana fibers obtained from leaves dipped in potassium permanganate 0.01% solution have shown extraordinary thermo-mechanical properties, such as tensile strength and thermal stability. This bio-suture material has potential strong properties, flexibility, and other mechanical properties that are similar to that of synthetic suture material currently found on the market. Agave americana leaf has strong properties of fibers.

In addition, Agave americana leaf natural cellulosic fibers are characterized by high moisture absorbency equal to 9%, low density of 1.36 gm/cm$^3$, and high tenacity in the range between 16-41 cN/tex. Besides, the Agave fibers found are flexible, smooth, and lustrous. The fibers contain about 73%-78% of the lignified form of cellulose.

The Agave americana leaf fiber surface is layered with a lignin layer. After the elimination of the lignin layer from the fiber surface, it becomes more flexible and smoother. The Agave fibers are very long with a mean length of up to 650 mm and an average linear density equal to 24 tex. The fiber of the Agave americana plant has an average length of up to 1.5m and a diameter of up to 4mm. The fiber length of Agaves americana fibers fall in the range of 1.43 m to 1.76 m. Meanwhile, the mean diameter of agave fibers varies between 367 $\mu$m and 411 $\mu$m. The linear density of each Agave fiber was calculated using the following formula:

$$\text{Linear density (Tex)} = \text{Weight} / \text{Length } X\ 100$$

Mechanical properties functional applicability of the newly developed bio suture material, on its mechanical properties, which should comply with the United States Pharmacopeia guidelines (2004) for the lowest tensile strength of the suture materials.

The bio-sutures from Agave americana fibers immersed in potassium permanganate (0.01%) solution displayed impressive mechanical properties that exceeded the US Pharmacopoeia's guidelines for minimum knot strength, which suggests that the bioabsorbable sutures will serve as an effective clinical counterpart to the synthetic sutures that are currently on the market. The suture material from Agave americana leaf has with high density, having approximately the following construction: U.S.P. sizes 0, 00, 3-0, 4-0, 5-0, and 6-0.

The Agave americana fibers coated by potassium permanganate are a good choice as a suture material because of their antibacterial activity properties.

The good elasticity of the novel bio-suture material, and the Agave fiber's ability to tie knots devoid of any sign of fracturing and breaking at the bends. Moreover, the Agave americana fibers coated by potassium permanganate are relatively thick as a result of the several cells that form the fiber bundle. Generally, Agave americana fibers coated by potassium permanganate are hydrophilic in nature and Agave fibers have properties of resistance to weak acids and alkalis. Agave americana fibers coated by potassium permanganate in a leaf contain different ensheathing cells including xylem and phloem. The cells are lignified to a greater or lesser degree and are hard in comparison with soft fibers.

The Agave americana fibers are difficult to extend. It means Agave fibers are rigid and have low elongation. The Agave leaf fiber is less cracked depending on the elongation and shows lower levels of deformations. Agave americana fibers are naturally occurring composites consisting mostly of cellulose fibrils embedded in a lignin matrix. The main elements of these fibers are cellulose, lignin, hemicellulose, and pectin.

## BRIEF DESCRIPTION OF THE DRAWINGS

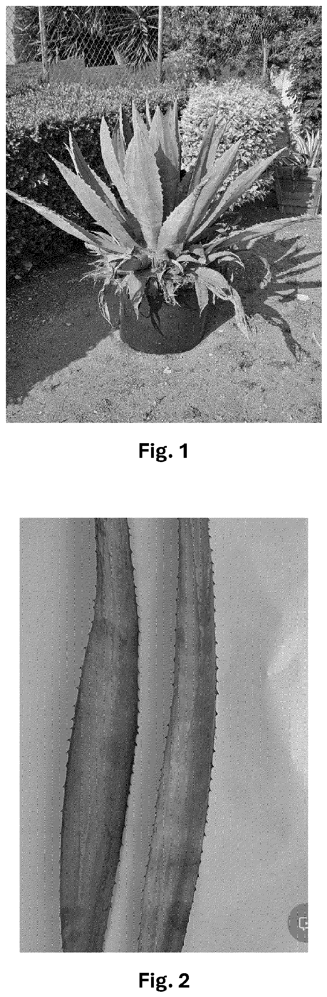

FIG. 1 is a picture showing the Agave americana plant.
FIG. 2 is a picture showing the Agave americana leaf.

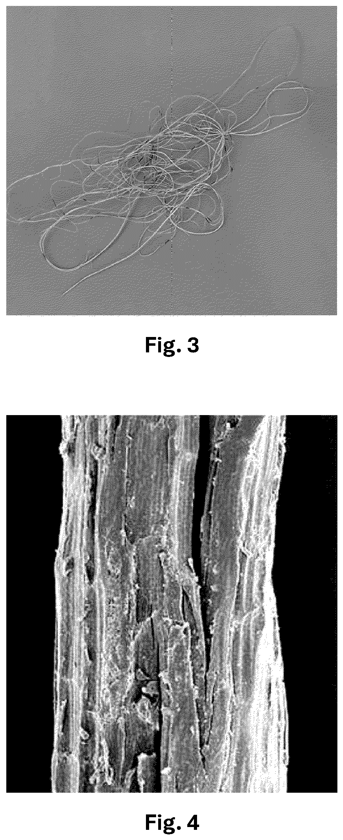

FIG. 3 is a picture showing the Agave americana leaf fibers.
FIG. 4 is a picture showing a scanning Electron Microscope micrograph of an Agave americana fiber.

## DETAILED DESCRIPTION

Detailed descriptions of the preferred embodiment are provided herein. It is to be understood, however, that the present inventions may be embodied in various forms. Therefore, specific details disclosed herein are not to be interpreted as limiting, but rather as a basis for the claims and as a representative basis for teaching one skilled in the art to employ the present inventions in virtually any appropriately detailed system, structure, or manner.

Various phases of extraction of Agave Americana fibers such as extraction, immersion, squashing, detaching, and final coating.

The fresh leaf of Agave Americana extracted from the plant. The leaf of Agave Americana is immersed in a closed water container for seven days to separate the fibers from the body of the leaf and achieve their extraction. The fibers are then squashed with a blunt instrument to remove the outer layer of the leaf and obtain the fibers. The leaf is subsequently immersed in water for three days at room temperature, then left to dry for two hours at temperatures up to 25 °C. The removal and detachment of the raw fibers into individual fibers could be done manually or with a machine.

To improve the antibacterial properties of Agave americana fibers, the fibers could undergo chemical and thermal sterilization treatment processes. The fibers of Agave Americana could be chemically cured by immersion with a Potassium permanganate (KMnO4) 0.1% solution. The 100 mg of Potassium permanganate (KMnO4) could be dissolved in one liter of distillate water to obtain a 1:10,000 (0.01%) solution. The dip coating method of Agave americana fibers is utilized by immersion with 0.1% Potassium permanganate solution.

Using the dip coating method with 0.1% Potassium permanganate solution the Agave fibers obtain several beneficial features such as less microbial growth and less scar tissue formation when the fibers are used for sutures.

The sterilization process is the procedure of killing and eliminating all types of microorganisms. The sterilization process could be achieved by using heat, radiation, chemicals, extreme pressure, and filtration. The Agave americana fiber bio-sutures must have "sterile" status and may be sterilized by gas such as Ethylene oxide gas. Ethylene oxide gas is commonly used to sterilize objects up to 60 °C with relative humidity above 30%. The Ethylene oxide gas penetrates through the medical-grade paper package to sterilize the sutures. Ethylene oxide gas can entirely kill all viruses, bacteria, bacterial spores, and fungi.

Sterile sutures are flushed with pure medical-grade nitrogen gas after removing Ethylene oxide gas from the suture materials.

Additionally, sutures from Agave americana leaf can be sterilized by a Sterilizing fluid containing Distilled Water, Isopropyl Alcohol, and Ethylene oxide gas. Agave americana fibers suture material can be sterilized by Gamma Radiation as well.

The Agave americana fibers may be mechanically combined with a surgical needle to create a surgical suture assembly. In these embodiments, one end of the agave suture may have been inserted into a drilled grove of the surgical needle and then crimped and pressed to create a permanent attachment.

In connection with the above criteria, the suture material from Agave americana leaf is one of the most innovative bio-suture materials.

In some embodiments, the surgical suture may comprise an elongated core covered by a plurality of bundles of bio-composites of fibers from an Agave americana plant leaf. The elongated core may comprise a main fiber or core fiber of an Agave americana plant leaf, having bundles of fibers or smaller individual fibers or bio-composites comprising fibers attached to it.

Accordingly, the present invention enables a healthcare provider to simplify and easily make a knot which needs a highly skill performance by a surgeon.

While a specific embodiment has been shown and described, many variations are possible. With time, additional features may be employed. The particular shape or configuration of the platform or the interior configuration may be changed to suit the system or equipment with which it is used.

Having described the invention in detail, those skilled in the art will appreciate that modifications may be made to the invention without departing from its spirit. Therefore, it is not intended that the scope of the invention be limited to the specific embodiment illustrated and described. Rather, it is intended that the scope of this invention be determined by the appended claims and their equivalents.

The Abstract of the Disclosure is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various embodiments for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus, the following claims are hereby incorporated into the Detailed Description, with each claim standing on its own as a separately claimed subject matter.

## Claims

1. A surgical suture assembly comprising:
   a surgical suture, the surgical suture comprising an elongated core covered by a plurality of bundles of bio-composites of fibers from an Agave americana plant leaf.

2. The surgical suture assembly of claim 1 further comprising a surgical needle mechanically connected to the plurality of the bundles of the bio-composites of the fibers made from the Agave americana plant leaf.

3. The surgical suture assembly of claim 1 wherein the plurality of the bundles of the bio-composites of the fibers made from the Agave americana plant leaf has a high density, approximately following U.S.P. sizes from 0, 00, 3-0, 4-0, 5-0 and 6-0.

4. The surgical suture of claim 1 further comprising a Potassium permanganate (KMnO4) 0.1% solution mechanically adhered to the surgical suture.

5. A method of manufacturing the surgical suture of claim 1 comprising:

   assembling the surgical suture; and
   chemically curing the surgical suture by immersion with a Potassium permanganate (KMnO4) 0.1% solution.

6. The method of claim 5 wherein the fibers of Agave americana is dip coated in the Potassium permanganate (KMnO4) 0.1% solution.

**Fig. 1**

**Fig. 2**

## Fig. 3

## Fig. 4

Europäisches
Patentamt

European
Patent Office

Office européen
des brevets

# EUROPEAN SEARCH REPORT

**Application Number**

EP 25 19 5556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SOLANKE SOHANI B ET AL: "Development and Evaluation of Surgical Sutures from Agave americana Linn. Var. Americana.", AMERICAN JOURNAL OF PHARMTECH RESEARCH, vol. 8, no. 6, 8 December 2018 (2018-12-08), pages 114-125, XP093346265, ISSN: 2249-3387, DOI: 10.46624/ajptr.2018.v8.i6.012 * page 3, line 3 - line 13 * * page 3, line 14 - line 15 * * abstract * | 1-6 | INV. A61L17/04 A61L17/14 |
| A | SNEHA K. R. ET AL: "Intrinsically radiopaque and antimicrobial cellulose based surgical sutures from mechanically powerful Agave sisalana plant leaf fibers", BIOMATERIALS SCIENCE, vol. 9, no. 23, 12 October 2021 (2021-10-12), pages 7944-7961, XP093346222, ISSN: 2047-4830, DOI: 10.1039/D1BM01316E * abstract; figure 1 * * page 11, left-hand column - right-hand column * * page 2, right-hand column, line 32 - line 44 * * page 2, left-hand column, line 36 - line 39 * | 1-6 | |

-----

-----

-/--

| | |
|---|---|
| TECHNICAL FIELDS SEARCHED (IPC) | |
| A61L | |

The present search report has been drawn up for all claims

| Place of search | Date of completion of the search | Examiner |
|---|---|---|
| The Hague | 12 December 2025 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 25 19 5556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUAMBO MARÍA PAULA ET AL: "Natural Cellulose Fibers for Surgical Suture Applications", POLYMERS, vol. 12, no. 12, 18 December 2020 (2020-12-18), page 3042, XP093258385, CH ISSN: 2073-4360, DOI: 10.3390/polym12123042 Retrieved from the Internet: URL:https://pmc.ncbi.nlm.nih.gov/articles/ PMC7765994/pdf/polymers-12-03042.pdf> * section 2.1 * * page 6, line 5 - line 6 * * table 2 * | 1-6 | |
| A | MADHU P ET AL: "A new study on effect of various chemical treatments on Agave Americana fiber for composite reinforcement: Physico-chemical, thermal, mechanical and morphological properties", POLYMER TESTING, ELSEVIER, AMSTERDAM, NL, vol. 85, 21 February 2020 (2020-02-21), XP086123716, ISSN: 0142-9418, DOI: 10.1016/J.POLYMERTESTING.2020.106437 [retrieved on 2020-02-21] * abstract * * page 2, left-hand column * | 4-6 | TECHNICAL FIELDS SEARCHED (IPC) |

The present search report has been drawn up for all claims

| Place of search | Date of completion of the search | Examiner |
|---|---|---|
| The Hague | 12 December 2025 | Lopez Serrano, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3716058 A
- US 9248580 B2
- US 9675341 B2
- US 10178991 B2
- US 10492780 B2
- US 11007296 B2
- US RE45426 E

- US 4259959 A
- US 9848868 B2
- US 8361112 B2
- US 7815662 B2
- US 8784438 B2
- WO 1992007519 A